# EUROPEAN PATENT SPECIFICATION

(11) **EP 0 539 601 B1**
(45) Date of publication and mention of the grant of the patent: **09.04.1997**
(21) Application number: 92909844.0
(22) Date of filing: 08.05.1992
(51) Int. Cl.: C07C 59/64, A61K 7/44, C07C 69/734, C09K 3/00

(54) **CINNAMIC ACID/GLYCEROL ADDUCT, ULTRAVIOLET ABSORBER, AND DERMATOLOGIC PREPARATION**
ZIMTSÄURE/GLYCEROL-ADDUKT, ULTRAVIOLETTABSORBER UND DERMATOLOGISCHE PRÄPARATION
PRODUIT D'ADDITION DE GLYCEROL/ACIDE CINNAMIQUE, ABSORBEUR D'ULTRAVIOLET ET PREPARATION DERMATOLOGIQUE

(30) Priority: 09.05.1991 JP 133488/91
(43) Date of publication of application: 05.05.1993
(73) Proprietor: SHISEIDO COMPANY LIMITED, Chuo-ku Tokyo 104-10 (JP)
(72) Inventor: KATO, Mikiko, Shiseido Laboratory 1050, Nippa-cho, Kanagawa 223 (JP); MIYAHARA, Reiji, Shiseido Laboratory, Kanagawa 223 (JP); UEHARA, Keiichi, Shiseido Laboratory, Kanagawa 223 (JP); TAKATA, Sadaki, Shiseido Laboratory, Kanagawa 223 (JP)
(74) Representative: Grünecker, Kinkeldey, Stockmair & Schwanhäusser Anwaltssozietät
(86) International application number: PCT/JP92/00587
(87) International publication number: WO 92/19592

(56) References cited:
- FR-M- 6 380
- JP-A- 2 003 638
- JP-A-58 110 535
- Tetrahedron Letters, 1970, no. 49, October 1970, Oxford, GB, W. OPPOLZER et al, "Intramolekulare Cycloadditionen von N-Alkyl-C-Phenoxymethyl-Nitronen an Ortho-staendige C=C-Doppelbindungen", pages 4313-4314
- CHEMICAL ABSTRACTS, vol. 106, no. 15, abstract no. 106(15) : 119400c, 1984, Columbus, Ohio, US, F. CLOUET et al, J. Chromatogr., 362(1), 125-31, 1986
- Chemical & Pharmaceutical Bulletin, vol. 39, no. 9, September 1991, Tokyo, JP, Y. SASHIDA et al, "Studies on the Chemical Constituents of the Bulbs of Lilium mackliniae", pages 2362-2368
- Encyclopedia of Chemistry, edited by the editor committee for Encyclopedia of Chemistry, vol. 2, page 55, left column, "oxycinnamic acid", vol. 3, page 50, right column, "Coumaric acid", vol. 3, page 51, left column, "Coumaric acid", and vol. 9, page 219, left column, "methoxy-cinnamic acid", 25 August 1962
- CHEMICAL ABSTRACTS, vol. 99, no. 6, abstract no. 99(6) : 43309u, 1983, Columbus, Ohio, US, R. ROELANDTS et al, Int. J. Dermatol., 22(4), 247-55
- CHEMICAL ABSTRACTS, vol. 107, no. 12, abstract no. 107(12) : 102417m, 1987, Columbus, Ohio, US, Y. Ibata, Fragrance J., 15(3), 54-8, 1987

## Description

This invention relates to an adduct of cinnamic acid and glycerin, an ultraviolet absorbent and an external preparation for skin and more particularly, a cinnamic acid derivative having ultraviolet absorbancy, and an ultraviolet absorbent and an external preparation for skin using the same.

Various influences of ultraviolet rays to skin, such as rapid aging of the skin, are recently well known. The ultraviolet rays included in sunlight are classified into long wavelength ultraviolet rays having a wavelength from 400 nm to 320 nm as UV-A, medium wavelength ultraviolet rays having a wavelength from 320 nm to 290 nm as UV-B, and short wavelength ultraviolet rays having a wavelength of not more than 290 nm as UV-C in a field of skin science.

Since most of the ultraviolet rays usually irradiating human body originate in sunlight, the UV-C are absorbed by the ozonosphere and the body is mainly influenced by the UV-A and UV-B.

Among these, the UV-B forms erythema or blister and causes aggravation of melanin formation and chromatosis when the skin is irradiated with the UV-B over a certain level.

In order to prevent aging of human skin and generation or increase of blots and flecks thereon, it is very important to protect human skin from the UV-B, and various types of UV-B absorbents have been developed.

The prior UV-B absorbents used for practical application include PABA derivatives, cinnamic acid derivatives salicylic acid derivatives, benzophenone derivatives, urocanine derivatives, camphor derivatives and a heterocyclic derivatives.

These types of UV-B absorbents are generally mixed in external preparations for skin such as cosmetics or quasi drugs.

The prior types of UV-B absorbents, however, are oil soluble in most cases and their solubility in water is very low, so their applications have been limited to a narrow area.

For example, JP-A-2-3638 discloses non-water soluble derivatives of cinnamic acid as UV-A-absorbents. As a water soluble UV-B absorbent, only 2-hydroxy-4-methoxy-5-sulfoxonium benzophenone sodium salt has been known, which however, has no sufficient ultraviolet ray absorptivity.

Accordingly, it is an object of the present invention to eliminate the above-described problems in the prior art and to provide a water soluble material having a high ultraviolet ray absorptivity.

As a result of studies undertaken by the inventors so as to attain this aim, it has been found that a new adduct of cinnamic acid and glycerin had an excellent ultraviolet ray absorptivity as well as good solubility in water. The present invention has been achieved on the bases of this finding.

The adduct of cinnamic acid and glycerin according to this invention is represented by the following general formula (I): 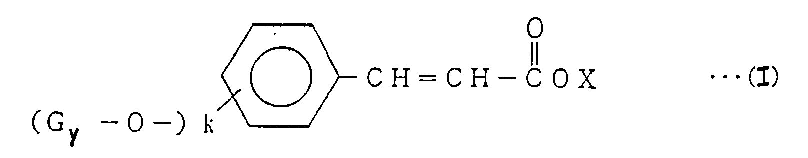 wherein
G represents a glycerin residue;
y represents the average mole number of glycerin residues and is at least 1;
k is 1 to 3; and
X represents hydrogen, an ion, a straight chain or branched chain alkyl group, an unsaturated alkyl group, a cycloalkyl group or G_{y};
provided that the compound of formula (I) is not 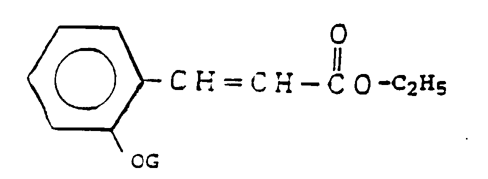 wherein G represents a glycerin residue.

Further, the present invention provides an ultraviolet absorbent, containing the above adduct of cinnamic acid and glycerin.

Additionally, the present invention provides a skin preparation containing one or more types of the above adduct of cinnamic acid and glycerin.

In the following the present invention will be explained in detail.

As stated above, G represents a glycerin residue, the bonding type of which is not limited; y represents the average mole number of glycerin residues and is at least 1. If the number of y becomes higher, the water solubility of the adduct becomes higher, but the ultraviolet absorptivity per weight becomes lower. Therefore, y is preferably in a range from 1 to 10.

X represents hydrogen; an ion such as sodium, potassium or ammonium; a straight chain alkyl group, a branched chain alkyl group, an unsaturated alkyl group and a cycloalkyl group, each having 1 or more, preferably 1 to 4, carbon atoms; or G_{y}. As examples of a methyl group, an ethyl group, an acetylenyl group, a propyl group, an isopropyl group, a propenyl group, a butyl group, an isobutyl group, a t-butyl group, and a butenyl group will be cited. Wavelength of absorbed ultraviolet rays does not differ remarkably in any case, but the methyl group and ethyl group are especially preferable because of their solubility in water and industrial applicability. If X is G_{y}, G and y are as defined above.

The adducts of cinnamic acid and glycerin described above are in solid or syrupy like state and are extremely excellent in their safety and stability. The adducts can be mixed in chemical products, such as dyes or inks, plastics, coating agents, and chemical fibers, and in addition can be mixed in pharmaceutical products, quasi drug products, cosmetics and cleaning agents as a component. Furthermore, the adduct has moisture holding ability.

The adduct of cinnamic acid and glycerin according to the present invention can be produced by general methods of glycerin addition reactions using an acidic catalyst or alkaline catalyst. Further, transesterification between polyglycerin and cinnamate can be applied.

For example, the following method can be applied.

The adduct can be synthesized by dissolving or suspending a compound represented by the following general formula (II), (wherein k is 1 to 3), in a non-aqueous solvent, such as dimethylsulfoxide, dimethylformamide, dioxane, dimethylacetamide, N-methylpyrrolidone, N-acetylmorpholine or N-methyl succinimide; by dissolving or suspending the compound and mixing the compound represented by the general formula (II) in acetone/aqueous solvent; or without solvent, and mixing the compound with glycidol in the presence of a catalyst at a temperature from 90 to 130°C. This reaction may be carried out under flow of a gas, such as N₂ or argon. Further one or more compounds represented by the general formula (II) may be used in this reaction.

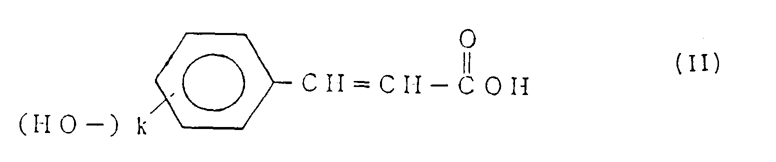

As examples of the catalyst applicable to this reaction, a Lewis acidic catalyst, such as BF₃·Et₂O and aluminum trichloride;an acidic catalyst, such as p-toluene sulfonic acid, hetero polyphosphoric acid, phosphoric acid, hydrochloric acid, and sulfuric acid; and an alkaline catalyst, such as sodium hydroxide, potassium hydroxide, potassium carbonate, sodium carbonate, sodium hydride, sodium alcoholate and sodium; will be cited.

The following method can also be applied.

The adduct can be synthesized by dissolving or suspending a compound represented by the following general formula (III) (wherein k is 1 to 3 and R₁ is a straight chain or branched chain alkyl group, an unsaturated alkyl group or a cycloalkyl group, each having 1 or more, preferably 1 to 4, carbon atoms) in a non-aqueous solvent, such as dimethylsulfoxide, dimethylformamide, dioxane, dimethylacetamide, N-methylpyrrolidone, N-acetylmorpholine or N-methyl succinimide, and mixing the compound with polyglycerin in the presence of a catalyst at a temperature from 90 to 130 °C. This reaction may be carried out under flow of a gas, such as N₂ or argon, or under reduced pressure conditions. Further one or more compounds represented by the general formula (III) may be used in this reaction.

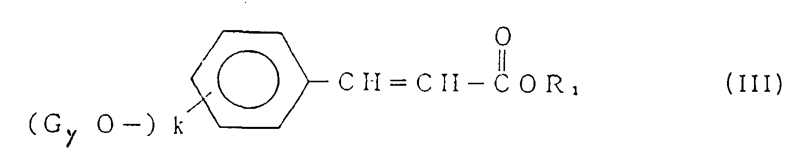

As examples of the catalyst applicable to this reaction, an acidic catalyst, such as p-toluene sulfonic acid, hetero polyphosphoric acid, phosphoric acid, hydrochloric acid, and sulfuric acid; an alkaline catalyst such as sodium hydroxide, potassium hydroxide, potassium carbonate, sodium carbonate, sodium hydride, sodium alcoholate and sodium; and a salt, such as ammonium chloride and sodium chloride; will be cited.

Further, the following method can be applied.

The adduct can be synthesized by dissolving or suspending a compound represented by the following general formula (IV) [(wherein k and R₁ are as-defined in formula (III)] in a non-aqueous solvent, such as dimethylsulfoxide, dimethylformamide, dioxane, dimethylacetamide, N-methylpyrrolidone, N-acetylmorpholine or N-methyl succinimide, and mixing the compound with glycidol in the presence of a catalyst at a temperature from 90 to 130°C. This reaction may be carried out under flow of a gas, such as N₂ gas or argon. Further, one or more compounds represented by the general formula (IV) may be used in this reaction. 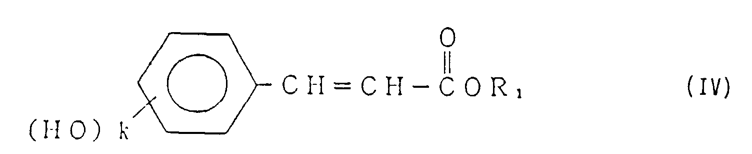 As examples of the catalyst applicable to this reaction, a Lewis acidic catalyst, such as BF₃·Et₂O and aluminum trichloride; an acidic catalyst, such as p-toluene sulfonic acid, hetero polyphosphoric acid, phosphoric acid, hydrochloric acid, and sulfuric acid; and an alkaline catalyst, such as sodium hydroxide, potassium hydroxide, potassium carbonate, sodium carbonate, sodium hydride, sodium alcoholate and sodium; will be cited.

After the reaction as described above, the solvent, glycerin and polymerized products of glycerin may be removed by distillation under reduced pressue, or the product may be used as it is.

In the resultant product obtained as described above, however, in addition to the adduct of cinnamic acid and glycerin represented by the general formula (I), salt, glycerin and polymerization products of glycerin are contained. In order to remove the glycerin, polymerized products or salt, the product may be purified by extraction with a solvent, such as methyl alcohol, ethyl alcohol, butyl alcohol, or isopropyl alcohol; or by distributing it in a mixture of water and methylethyl ketone or n-butanol having a large volume of salt therein and fractionating the organic solvent layer. Also in order to remove glycerin or polymerized products thereof for isolating the compound, the resultant product may be purified by column chromatography by suspending the resultant product in water or a mixture of water and alcohol, applying it in a opposite phase partition column, such as hyper porous polymer (such as HIPOROUS RESIN supplied from MITSUBISHI KASEI KOGYO KABUSHIKI KAISHA) or octadecyl silica, and then using water as a first eluent and a mixed solution of water with a polar organic solvent, such as an alcohol, e.g. methanol or ethanol, or acetonitrile as a second eluent, and fractionating and taking out this eluent. Also the resultant product can be purified by recrystallization.

The adducts of cinnamic acid and glycerin thus obtained are excellent in their resistance to oxidation and chemical stability. As the adduct has a glycerin group, the adduct has low stimulus for skin or eye. So the adduct of cinnamic acid and glycerin can be mixed in chemical products, such as paints, inks, plastics, coating agents and chemical fibers.

As the adducts of cinnamic acid and glycerin according to the present invention are excellent in their safety, the compounds can be mixed in cosmetics or pharmaceutical drugs. In addition to the embodiments of the present invention, the adducts can appropriately be mixed with other components for cosmetics or pharmaceutical drugs. As examples of such a component, oily components, such hydrocarbons, fats and oils, e.g. liquid paraffin, squalane, vaseline, cetyl alcohol, isostearyl alcohol, cetyl 2-ethylhexanoate, 2-octyldodecyl alcohol, glycerin triisostearate, Macademian nuts oil, and lanolin; waxes, silicones, surfactants, thickeners, neutralizers, antiseptics, germicides, anti-oxidants, powder components, pigments, perfumes, other ultraviolet absorbents, drugs, metallic sealants and pH modifiers, will be cited.

The adducts of cinnamic acid and glycerin thus obtained are excellent in their chemical stability and resistance to oxidation, and are also water soluble and can absorb UV-B. Furthermore they are excellent in moisture holding properties.

In the following the drawings are briefly described.
FIG. 1 is a gas chromatogram of an adduct of cinnamic acid and glycerin (mixture) according to the example 1 of the present invention;
FIG. 2 is a gas chromatogram of glyceryl p-glyceroxy cinnamate (adduct of 2 mole glycerin) according to the example 1 of the present invention;
FIG. 3 is an infrared absorption spectrum of glyceryl p-glyceroxy cinnamate (adduct of 2 mole glycerin) according to the example 1 of the present invention;
FIG. 4 is a ¹³C-NMR spectrum of glyceryl p-glyceroxy cinnamate (adduct of 2 mole glycerin) according to the example 1 of the present invention;
FIG. 5 is a ¹H-NMR spectrum of glyceryl p-glyceroxy cinnamate (adduct of 2 mole glycerin) according to the example 1 of the present invention;
FIG. 6 is an ultraviolet absorption spectrum of glyceryl p-glyceroxy cinnamate (adduct of 2 mole glycerin) according to the example 1 of the present invention;
FIG. 7 is a gas chromatogram of glyceryl p-glyceroxy cinnamate (adduct of 3 mole glycerin) according to the example 1 of the present invention;
FIG. 8 is a ¹³C-NMR spectrum of glyceryl p-glyceroxy cinnamate (adduct of 3 mole glycerin) according to the example 1 of the present invention;
FIG. 9 is an infrared absorption spectrum of glyceryl p-glyceroxy cinnamate (adduct of 3 mole glycerin) according to the example 4 of the present invention;
FIG. 10 is a ¹³C-NMR spectrum of glyceryl p-glyceroxy cinnamate (adduct of 3 mole glycerin) according to the example 4 of the present invention;
FIG. 11 is a ¹H-NMR spectrum of glyceryl p-glyceroxy cinnamate (adduct of 3 mole glycerin) according to the example 4 of the present invention;
FIG. 12 is an ultraviolet absorption spectrum of glyceryl p-glyceroxy cinnamate (adduct of 3 mole glycerin) according to the example 4 of the present invention;

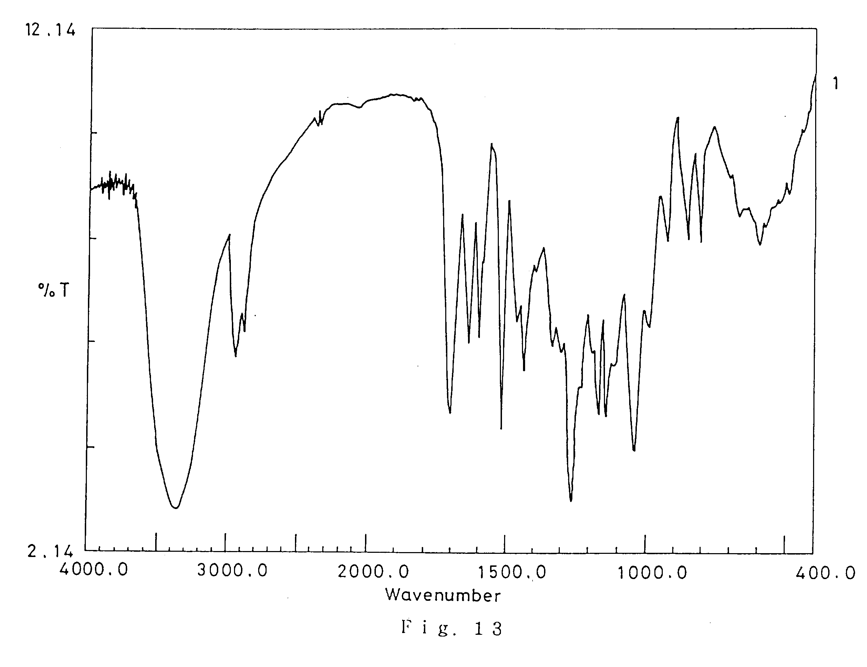

FIG. 13 is an infrared absorption spectrum of glyceroxy cinnamate according to the example 8 of the present invention;
FIG. 14 is an infrared absorption spectrum of glyceryl m,p-glyceroxy cinnamate (adduct of 3 mole glycerin) according to the example 9 of the present invention;
FIG. 15 is a ¹³C-NMR spectrum of glyceryl m,p-glyceroxy cinnamate (adduct of 3 mole glycerin) according to the example 9 of the present invention; and
FIG. 16 is a ¹H-NMR spectrum of glyceryl m,p-glyceroxy cinnamate (adduct of 3 mole glycerin) according to the example 9 of the present invention.

The embodiments of the present invention will be described hereinafter. It should be noted that the embodiments are not intended to limit the present invention. The mixing rates are given in weight %.

### Example 1 Preparation for the adduct of cinnamic acid and glycerin (1) according to a reaction using an acidic catalyst

8.719 g of p-hydroxy cinnamic acid were dissolved in 10 ml of DMSO, and 19.7 g of glycidol were added. The mixture was stirred and heated to 90 °C. A catalytic amount of boron trifluoride ethyl ether was added. Heating and agitation was carried out for 2 hours and then the adduct of cinnamic acid and glycerin was obtained.

The reaction system was fractionated by column chromatography using the hyperporous polymer (HIPOROUS RESIN supplied from MITSUBISHI KASEI KOGYO KABUSHIKI KAISHA), distilled water as a first eluent and a mixture of ethyl alcohol and distilled water with the mixing ratio of 3:7 as second eluent. The fraction of which the mixing ratio of ethyl alcohol and distilled water was 3:7 was evaporated and the adduct of cinnamic acid and glycerin was purified.

The yield of the adduct of cinnamic acid and glycerin was 8.9g.

0.1 ml of trimethylsilylchlorosilane, 0.1 ml of trimethylsilylimidazole and N,0-bis(trimethylsilyl)acetamide were added to 10 mg of the adduct, heated at 80 °C for 30 minutes and the resultant TMS compound was obtained. This TMS compound was applied to gas chromatography (CHROMATOPACK GC-9A supplied from SHIMAZU CORPORATION, COLUMN; DIASOLID ZT made of glass and having 3φ × 50cm supplied from GASCHRO KOGYO CORPORATION, FLOW RATE; 60 ml/min. N₂, DETECTOR; FID, TEMPERATURE UP; 100 °C to 340 °C 10 °C/min., TEMPARATURE OF SAMPLE GASIFICATION ROOM; 360°C) and then analyzed.

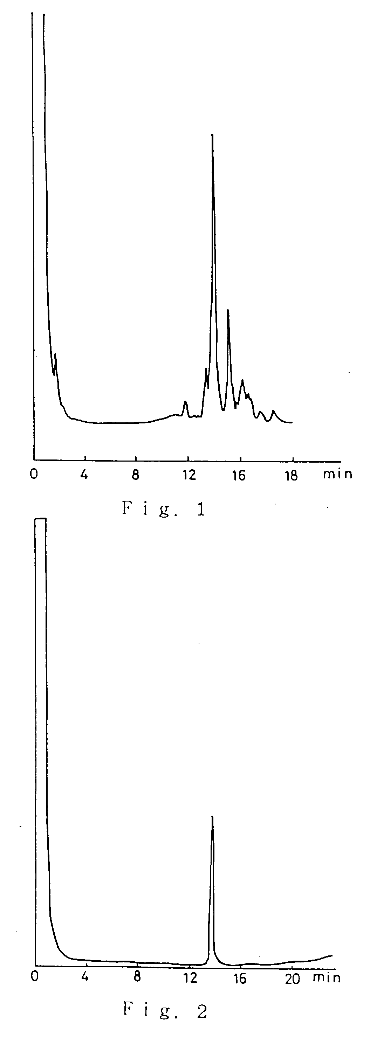

The gas chromatogram is shown in Fig. 1.

It will be understood from the Fig.1 that the adduct of cinnamic acid and glycerin according to the example 1 is a mixture of several compounds. The main components were separated and identified. As a result of this separation and identification, following compounds (1), (2) were identified.

### (1) Adduct of 2 mole glycerin

(y=1, k=1 and X=G in the formula I)

The adduct of 2 mole glycerin represented by the structural formula V described below was identified in the mixture of adducts manufactured by the example 1.

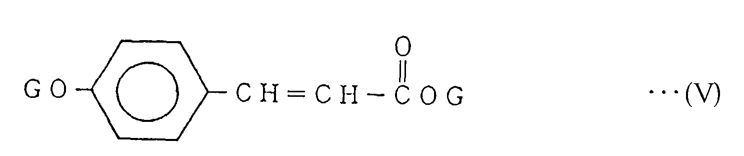

Namely, the compound which had a retention time of 13.9 minutes was isolated and purified by silica gel column chromatography (eluent; chloroform to chloroform/methanol = 10:1 (v/v)), and analyzed by methods ① to ⑧. The obtained compound was solid. This compound was treated as the sample 1.

### ① GAS CHROMATOGRAPHY

0.1 ml of trimethylsilylchlorosilane, 0.1 ml of trimethylsilylimidazol and N,0-bis (trimethylsilyl) acetamide were added to 10 mg of the adduct, and heated at 80 °C for 30 minutes. Thus, the resultant TMS compound was obtained. This TMS compound was subjected to gas chromatography (CHROMATOPACK GC-9A supplied from SHIMAZU CORPORATION, COLUMN; DIASOLID ZT made of glass and having 3φ × 50cm supplied from GASCHRO KOGYO CORPORATION, FLOW RATE; 60 ml/min. N₂, DETECTOR; FID, TEMPERATURE UP; 100 TO 340°C 10°C/min., TEMPARATURE OF SAMPLE GASIFICATION ROOM; 360°C) and then analyzed.

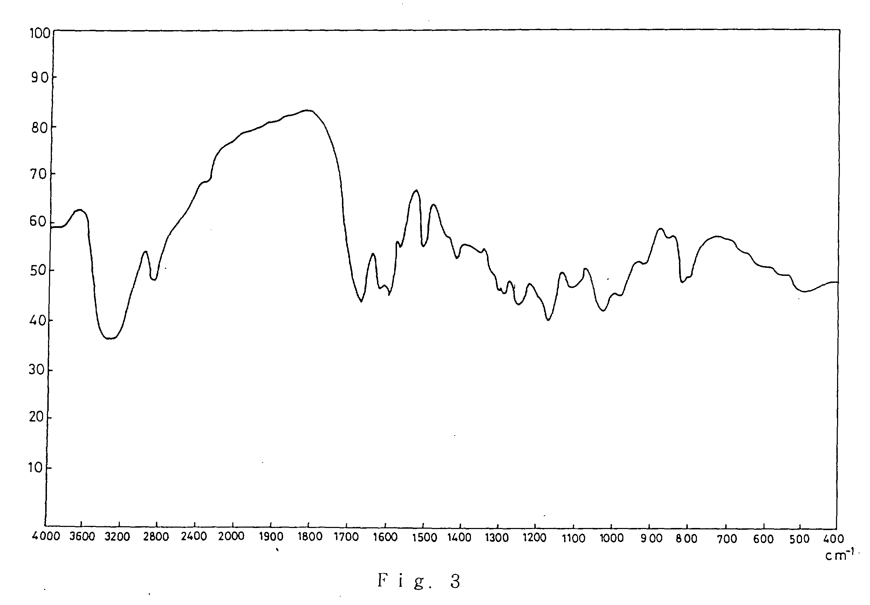

The gas chromatogram is shown in Fig. 2.

### ② INFRARED ABSORPTION SPECTROSCOPY

The infrared absorption spectrum was measured by using a IRA-1 infrared absorption spectrometer supplied from NIHON BUNKO KABUSHIKI KAISHA with KBr disk. The absorption due to the stretching vibration of the hydroxyl group at 3350 cm⁻¹, the stretching vibration of the glyceroxy group at 2900 cm⁻¹ and the stretching vibration of the carbonyl group at 1690 cm⁻¹ were observed.

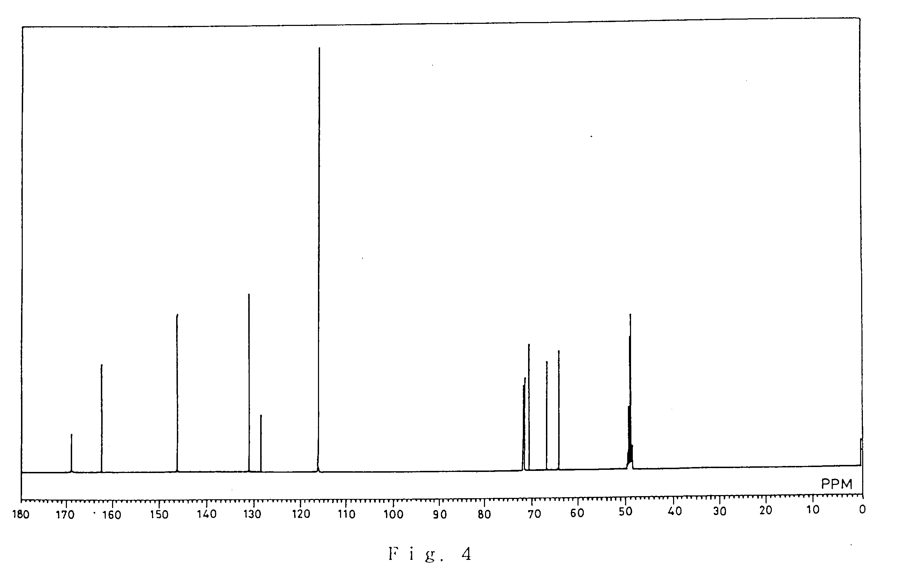

The result is shown in FIG. 3.

### ③ ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the cinnamate part were observed at 169 ppm, 162 ppm, 146 ppm, 131 ppm, 128 ppm, and 116 ppm, and signals from carbon atoms in the glyceryl part were observed at 72 ppm, 71 ppm, 70 ppm, 67 ppm and 64 ppm.

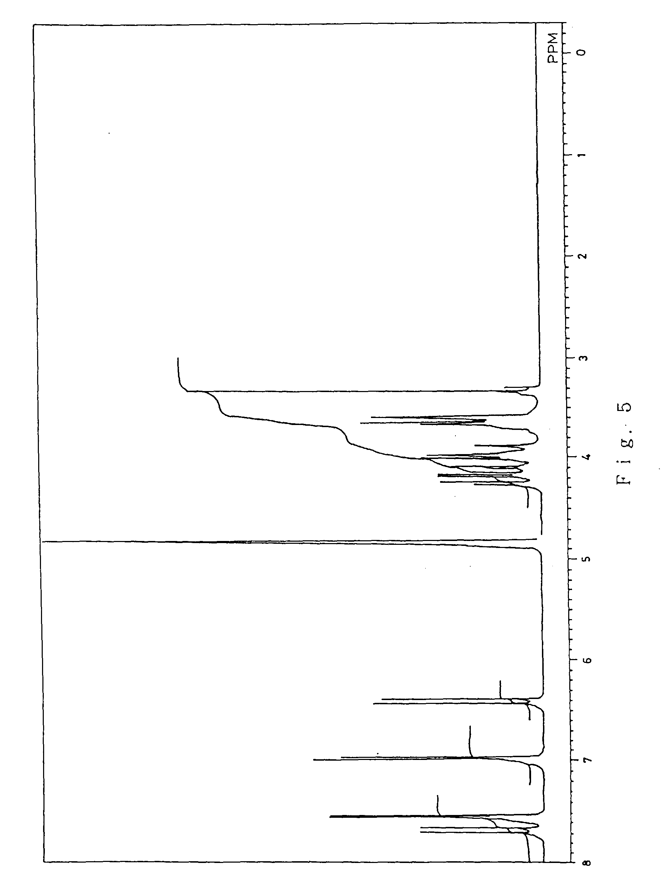

The result is shown in FIG. 4.

### ④ ¹H-NMR SPECTROSCOPY

The ¹H-NMR spectrum was measured by using CD₃OD as a solvent with JOEL GX-400 supplied from NIPPON DENSHI KABUSHIKI KAISHA at room temperature. Signals from hydrogen atoms in the cinnamic acid part were observed at δ 7.67 (1H, d, J=16 Hz), 7.54 (2H, d, J=8.8 Hz), 7.00 (2H, d, J=8.8 Hz) and 6.40 (1H, d, J=16.1 Hz) , and signals from hydrogen atoms in the glyceryl group were observed in a range from δ 4.28 to 3.58 ppm.

The result is shown in FIG. 5.

### ⑤ ULTRAVIOLET RAY ABSORPTION SPECTROSCOPY

The ultraviolet ray absorption spectrum was measured by using the UVIDEC 610 C ultraviolet ray absorption spectrometer from NIHON BUNKO KOGYO KABUSHIKI KAISHA with methanol as a solvent. The peak absorptions were observed at 221.4 nm and 309.6 nm.

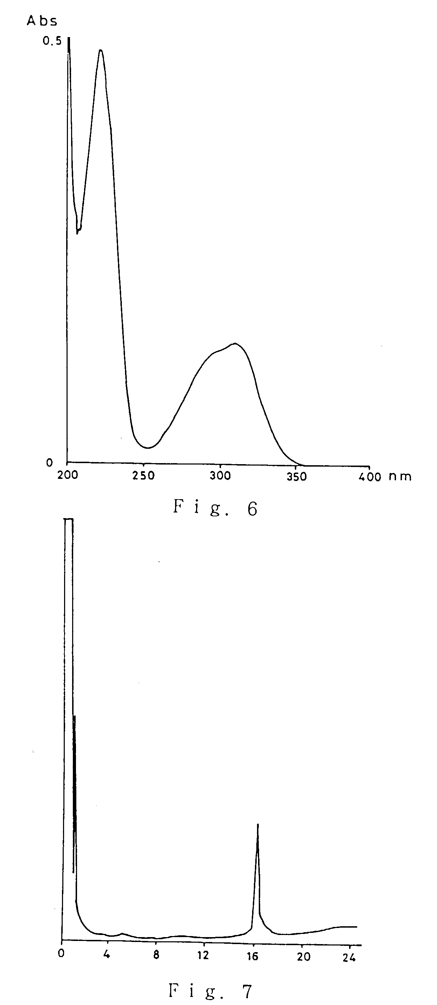

The result is shown in FIG.6.

### ⑥ MELTING POINT

The melting point was measured by using a capillary type melting point measuring apparatus supplied from ARTHUR H. THOMS COMPANY. The melting point was in a range from 116 to 123°C.

### ⑦ PHENOL INDICATION

The product was spotted on TLC, a phenol indicator was sprayed thereon with a sprayer. And a 10 % sodium bicarbonate solution was sprayed over it. A coloration indicating presence of phenol was not observed.

### ⑧ BCG INDICATION

The product was spotted on TLC, and a BCG indicator was sprayed thereon with a sprayer. A coloration was not observed.

### (2) Adduct of 3 mole glycerin

(y+y=3, k=1 in the general formula I)

The adduct of 3 mole glycerin represented by the structural formula (VI) described below was identified in the mixture of adducts manufactured by the example 1.

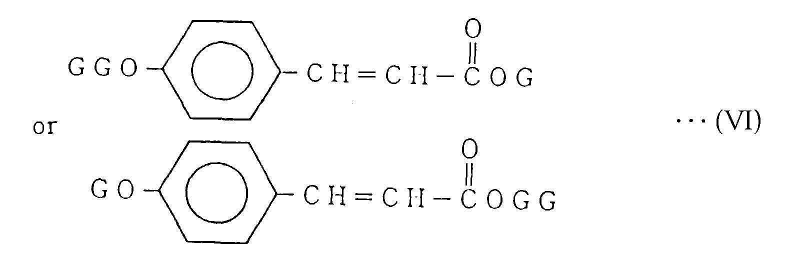

Namely, the compound which had a retention time of 16.3 minutes was isolated and purified by silica gel column chromatography (eluent; chloroform to chloroform/methanol = 10:1 (v/v)), and analyzed by methods ① to ④. The obtained compound was solid. This compound was treated as the sample 2.

### ① GAS CHROMATOGRAPHY

0.1 ml of trimethylsilylchlorosilane, 0.1 ml of trimethylsilylimidazol and N,O-bis (trimethylsilyl) acetamide were added to 10 mg of the adduct, and heated at 80 °C for 30 minutes. The resultant TMS compound was obtained. This TMS compound was subjected to gas chromatography (CHROMATOPACK GC-9A supplied from SHIMAZU CORPORATION, COLUMN; DIASOLID ZT made of glass and having 3φ × 50cm supplied from GASCHRO KOGYO CORPORATION, FLOW RATE; 60 ml/min. N₂, DETECTOR; FID, TEMPERATURE UP; 100 TO 340 °C 10°C/min., TEMPARATURE OF SAMPLE GASIFICATION ROOM; 360°C) and then analyzed.

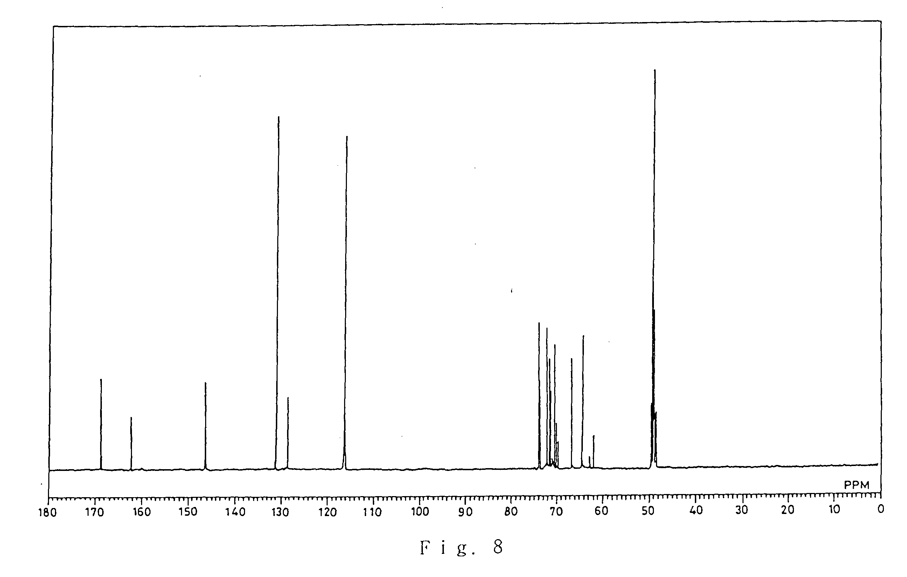

The gas chromatogram is shown in Fig. 7.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the cinnamate part were observed at 169 ppm, 162 ppm, 146 ppm, 131 ppm, 128 ppm, and 116 ppm, and signals from carbon atoms in the glyceryl group were observed at 72 ppm, 71 ppm, 70 ppm, 67 ppm and 64 ppm.

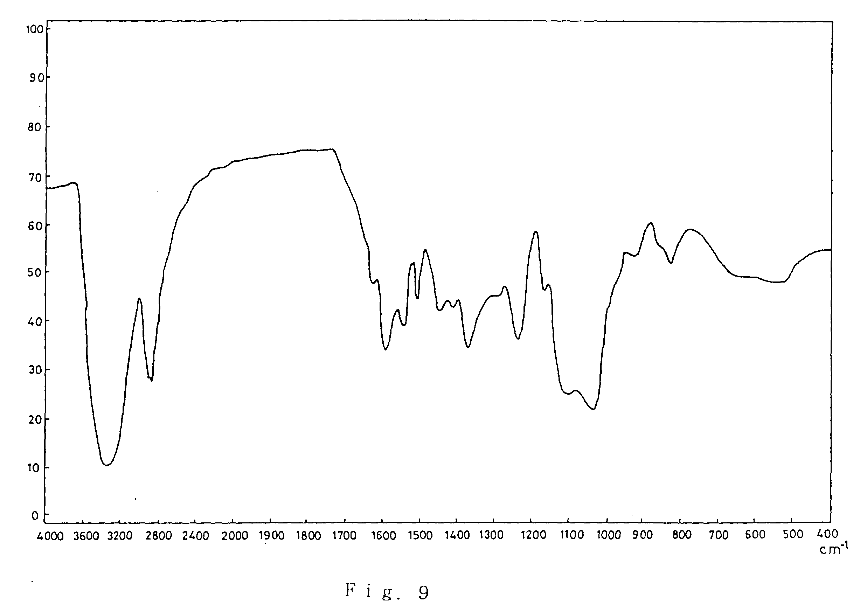

The result is shown in FIG. 8.

### ③ PHENOL INDICATION

The product was spotted on TLC, a phenol indicator was sprayed thereon with a sprayer and a 10 % sodium bicarbonate solution was sprayed over it. A coloration indicating presence of phenol was not observed.

### ④ BCG INDICATION

The product was spotted on TLC, and a BCG indicator was sprayed thereon with a sprayer. A coloration was not observed.

### Example 2 Preparation of the adduct of cinnamic acid and glycerin (2) according to a reaction using an acidic catalyst

17.4 g of p-hydroxy cinnamic acid and 40.0 g of glycidol were dissolved in 20 ml of DMSO, and a catalytic amount of sulfuric acid was added. The solution was heated to 90 °C and then heating and agitation was carried out for 3 hours. The reaction mixture was neutralized by adding sodium hydroxide and then an adduct of cinnamic acid and glycerin was obtained.

The reaction system was fractionated by column chromatography using the hyperporous polymer (HIPOROUS RESIN supplied from MITSUBISHI KASEI KOGYO KABUSHIKI KAISHA), distilled water as a first eluent and a mixture of ethyl alcohol and distilled water with the mixing ratio of 3:7 as a second eluent. The effluent fraction of which the mixing ratio of ethyl alcohol and distilled water was 3:7 was evaporated and the adduct of cinnamic acid and glycerin was purified.

The yield of the adduct of cinnamic acid and glycerin was 18 g.

### Example 3 Preparation of the adduct of cinnamic acid and glycerin (3) according to a reaction using an acidic catalyst

1.74 g of p-hydroxy cinnamic acid and 4.0 g of glycidol were dissolved in 2 ml of DMSO, and a catalytic amount of hydrochloric acid was added. The solution was heated to 90 °C and heating and agitation were carried out for 1 hour. The reaction mixture was neutralized by adding sodium hydroxide and then the adduct of cinnamic acid and glycerin was obtained.

The reaction system was fractionated by column chromatography using the hyperporous polymer (HIPOROUS RESIN supplied from MITSUBISHI KASEI KOGYO KABUSHIKI KAISHA), distilled water as a first eluent and a mixture of ethyl alcohol and distilled water with the mixing ratio of 3:7 as a second eluent. The effluent fraction of which the mixing ratio of ethyl alcohol and distilled water was 3:7 was evaporated and the adduct of cinnamic acid and glycerin was purified.

The yield of the adduct of cinnamic acid and glycerin was 2 g.

### Example 4 Preparation of the adduct of cinnamic acid and glycerin (1) according to a reaction using an alkaline catalyst

8.719 g of p-hydroxy cinnamic acid were dissolved in 10 ml of DMSO, and 100 mg of sodium hydroxide were added. The mixture was stirred and heated to 90 °C under a flow of N₂ gas. 19.7 g of glycidol were added gradually and heating and agitation was carried out for 2 hours. The reaction mixture was then neutralized by adding hydrochloric acid and then the adduct of cinnamic acid and glycerin was obtained.

The reaction system was fractionated by the column chromatography using the hyperporous polymer (HIPOROUS RESIN supplied from MITSUBISHI KASEI KOGYO KABUSHIKI KAISHA), distilled water as a first eluent and a mixture of ethyl alcohol and distilled water with the mixing ratio of 3:7 as a second eluent. The fraction of which the mixing ratio of ethyl alcohol and distilled water was 3:7 was evaporated and the adduct of cinnamic acid and glycerin was purified.

The yield of the adduct of cinnamic acid and glycerin was 13.5 g.

### (1) Sodium glyceroxy cinnamate

(y=3, k=1 and X=Na)

The adduct of 3 mole glycerin represented by the structural formula (VII) described below was identified in the mixture of adducts manufactured by the example 4.

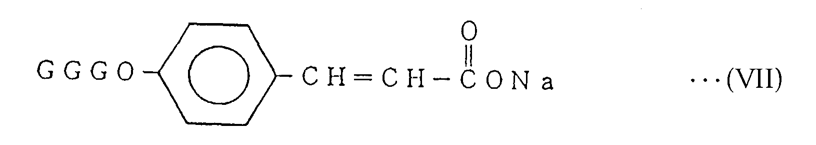

The compound which was synthesized in example 4 was isolated and purified by silica gel column chromatography (eluent; chloroform to chloroform/methanol = 10/1 (v/v)), and the obtained solid compound was analyzed by methods ① to ⑤, This compound was treated as sample 3.

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900 cm⁻¹ and the stretching vibration of the carbonyl group at 1690 cm⁻¹ were observed.

The result is shown in FIG. 9.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the cinnamate ester part were observed at 169 ppm, 162 ppm, 146 ppm, 131 ppm, 128 ppm, 118 ppm and 116 ppm, and signals from carbon atoms in the glyceryl group were observed in a range from 72 ppm to 64 ppm.

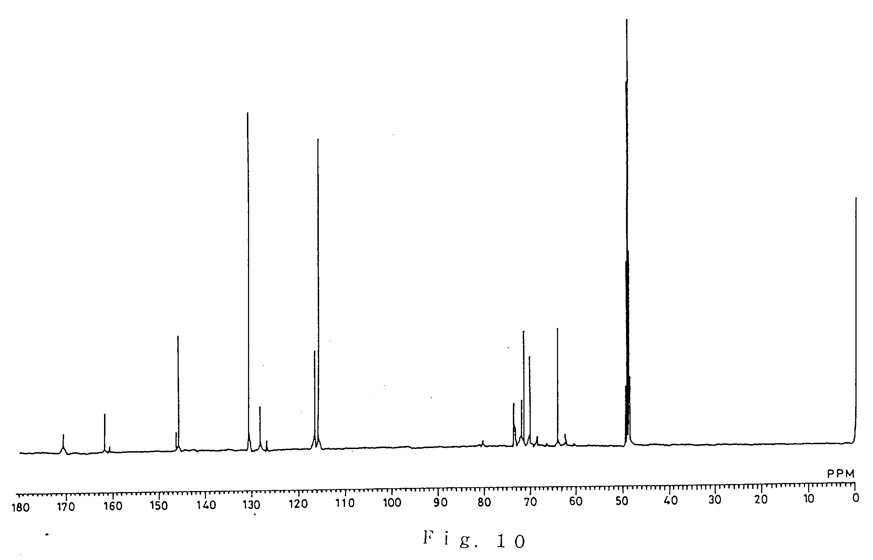

The result is shown in FIG. 10.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at room temperature. Signals from hydrogen atoms in the cinnamic acid part were observed at δ 7.67 (1H, d, J=16 Hz), 7.54 (2H, d, J=8.8 Hz), 7.00 (2H, d, J=8.8 Hz) and 6.40 (1H, d, J=16.1 Hz), and signals from hydrogen atoms in the glyceryl group were observed in a range from δ 5.0 to 3.2 ppm.

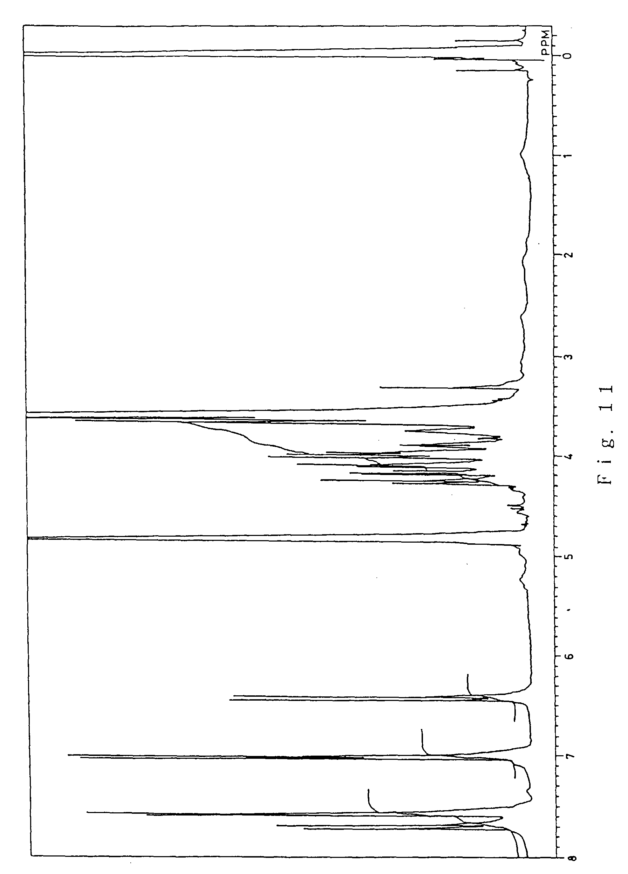

The result is shown in FIG. 11.

### ④ ULTRAVIOLET RAY ABSORPTION SPECTROSCOPY

The ultraviolet ray absorption spectrum was measured by using a UVIDEC 610 C ultraviolet ray absorption spectrometer from NIHON BUNKO KOGYO KABUSHIKI KAISHA with methanol as a solvent. The peak absorptions were observed at 222.4 nm and 287.6 nm.

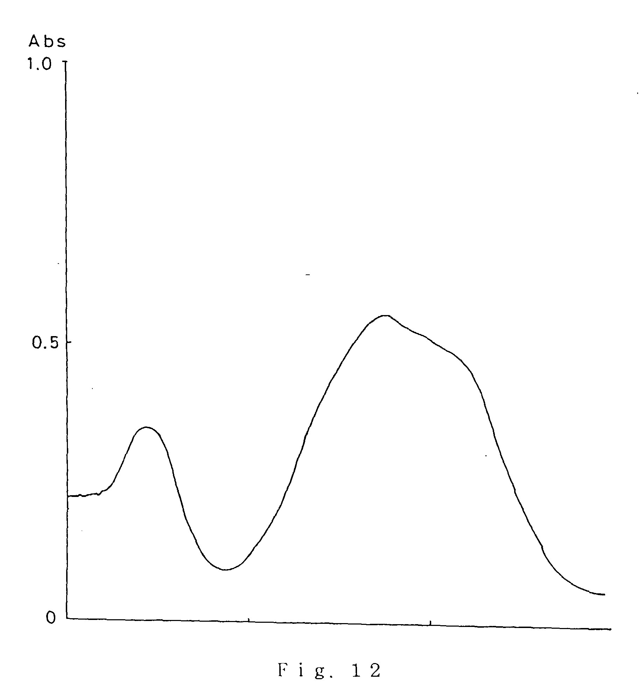

The result is shown in FIG. 12.

### ⑤ PHENOL INDICATION

The product was spotted on TLC, a phenol indicator was sprayed thereon with a sprayer and a 10 % sodium bicarbonate solution was sprayed over it. A coloration indicating presence of phenol was not observed.

### Example 5 Preparation of the adduct of cinnamic acid and glycerin (2) according to a reaction using an alkaline catalyst

4.35 g of p-hydroxy cinnamic acid were dissolved in 5 ml of DMSO, and 50 mg of potassium hydroxide were added. The mixture was stirred and heated to 90 °C under flow of N₂ gas. 10.0 g of glycidol were added gradually and heating and agitation was carried out for 1.5 hours. The reaction mixture was neutralized by adding hydrochloric acid and the adduct of cinnamic acid and glycerin was obtained.

The reaction system was fractionated by the column chromatography using the hyperporous polymer (HIPOROUS RESIN supplied from MITSUBISHI KASEI KOGYO KABUSHIKI KAISHA), distilled water as a first eluent and a mixture of ethyl alcohol and distilled water with the mixing ratio of 3:7 as a second eluent. The effluent fraction of which the mixing ratio of ethyl alcohol and distilled water was 3:7 was evaporated and adduct of cinnamic acid and glycerin.

The yield of the adduct of cinnamic acid and glycerin was 7.1 g.

### Example 6 Preparation of the adduct of cinnamic acid and glycerin (3) according to a reaction using an alkaline catalyst

2.8 g of p-hydroxy cinnamic acid were dissolved in 3 ml of DMSO, and 10 mg of sodium were added. The mixture was stirred and heated to 90 °C under a flow of N₂ gas. 5.0 g of glycidol were added gradually and heating and agitation were carried out for 1 hour. The reaction mixture was neutralized by adding hydrochloric acid and then the adduct of cinnamic acid and glycerin was obtained.

The reaction system was fractionated by the column chromatography using the hyperporous polymer (HIPOROUS RESIN supplied from MITSUBISHI KASEI KOGYO KABUSHIKI KAISHA), distilled water as a first eluent and a mixture of ethyl alcohol and distilled water with the mixing ratio of 3:7 as a second eluent. The effluent fraction of which the mixing ratio of ethyl alcohol and distilled water was 3:7 was evaporated and the adduct of cinnamic acid and glycerin was purified.

### Example 7 Preparation of the adduct of cinnamic acid and glycerin (4) according to a reaction using an alkaline catalyst

2.8 g of p-hydroxy cinnamic acid were dissolved in 3 ml of DMSO, and 10 mg of sodium hydride of which the oil was washed by hexane were added. The mixture was stirred and heated to 90 °C under a flow of N₂ gas. 5.0 g of glycidol were added gradually and heating and agitation were carried out for 1.5 hours. The reaction mixture was neutralized by adding hydrochloric acid and then the adduct of cinnamic acid and glycerin was obtained.

The reaction system was fractionated by the column chromatography using the hyperporous polymer (HIPOROUS RESIN supplied from MITSUBISHI KASEI KOGYO KABUSHIKI KAISHA), distilled water as a first eluent and a mixture of ethyl alcohol and distilled water with the mixing ratio of 3:7 as a second eluent. The effluent fraction of which the mixing ratio of ethyl alcohol and distilled water was 3:7 was evaporated and the adduct of cinnamic acid and glycerin was purified.

The yield of the adduct of cinnamic acid and glycerin was 4.3 g.

### Example 8 Methyl P-Glyceroxy cinnamate

100 mg of p-glyceroxy cinnamic acid were dissolved in 3 ml of methanol. 1 ml of sodium methylate was added and the mixture was stirred at room temperature for 30 minutes. The system was neutralized by adding HCl-MeOH, and then the glyceroxy cinnamate was obtained.

The compound which was evaporated under reduced pressure conditions was isolated and purified by silica gel column chromatography using chloroform-methanol as an eluent, and then a solid glyceroxy cinnamate was obtained.

The yield of the glyceroxy cinnamate was 60 mg.

The compound described above was analyzed by methods ① and ②. This compound was treated as sample 5.

### ① INFRARED ABSORPTION SPECTROSCOPY

The infrared absorption spectrum was measured by using the IRA-1 infrared absorption spectrometer supplied from NIHON BUNKO KABUSHIKI KAISHA with KBr disk. Absorptions due to the stretching vibration of the hydroxyl group at 3350 cm⁻¹, the stretching vibration of the glyceroxy group at 2900 cm⁻¹ and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the cinnamate part were observed at 169 ppm, 162 ppm, 146 ppm, 131 ppm, 128 ppm and 116 ppm, and signals from carbon atoms in the glyceryl group were observed in a range from 72 ppm to 64 ppm.

### Example 9 Glyceryl m,p-diglyceroxy cinnamate

3.0 g (16.7 mmol) of caffeic acid and 9.9 g (133 mmol) of glycidol were dissolved in 5 ml of DMSO, and the mixture was stirred and heated to 90 °C. A catalytic amount of BF₃.Et₂O was added and heating and agitation were carried out for 3 hours. The system was cooled by standing in air, and water in the system was removed under conditions of reduced pressure. The residue was fractionated by the chromatography using the column, DIAION HP20 (MITSUBISHI KASEI KABUSHIKI KAISHA), distilled water as a first eluent and a mixture of EtOH/H₂O=3/7(v/v) as a second eluent. The effluent fraction of which the mixture of EtOH/H₂O=3/7 was evaporated and glyceryl m,p-diglyceroxy cinnamate was obtained. The results of analysis of the resultant glyceryl m,p-diglyceroxy cinnamate were indicated below.

### ① INFRARED ABSORPTION SPECTROSCOPY

The infrared absorption spectrum was measured by using the IRA-1 infrared absorption spectrometer supplied from NIHON BUNKO KABUSHIKI KAISHA with KBr disk. Absorptions due to the stretching vibration of the bond between carbon and oxygen at 3375 cm⁻¹, the stretching vibration of the bond between carbon and hydrogen at 2940cm⁻¹ and the stretching vibration of the carbonyl group at 1700 cm⁻¹ were observed.

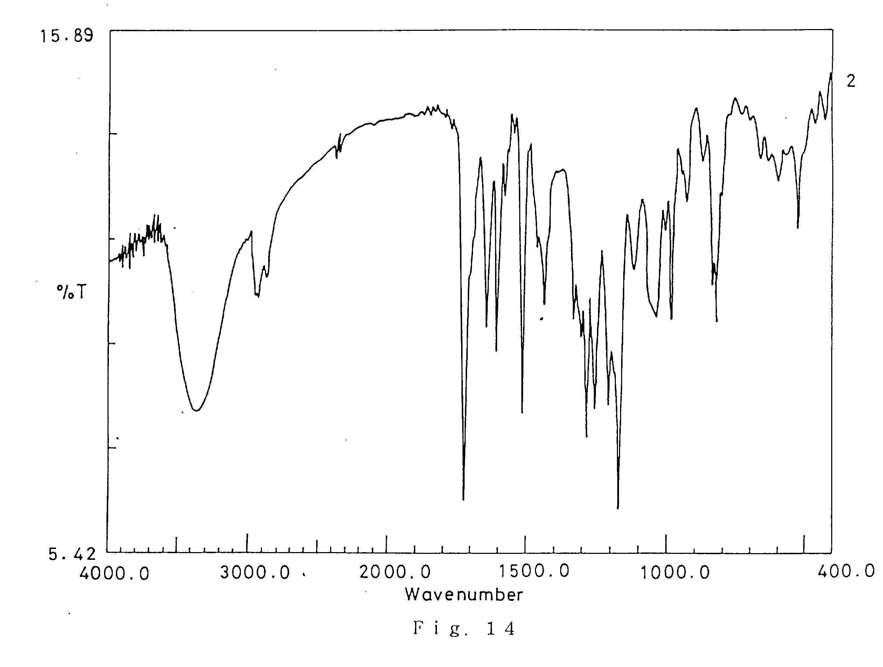

The result is shown in the Fig. 14.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the caffeic acid part were observed in a range from 160 ppm to 103 ppm, and signals from carbon atoms in the glyceryl group were observed in a range from 64 ppm to 43 ppm.

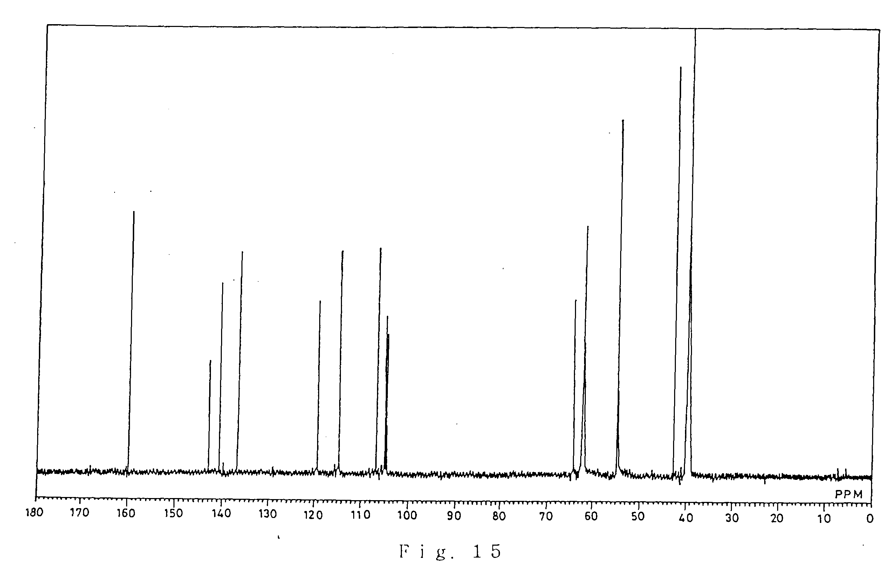

The result is shown in the Fig. 15.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at room temperature. Signals from hydrogen atoms in the caffeic acid part were observed in a range from δ 7.62 to 6.374 ppm, and signals from hydrogen atoms in the glyceryl group were observed in a range from 6 4.14 to 3.34 ppm.

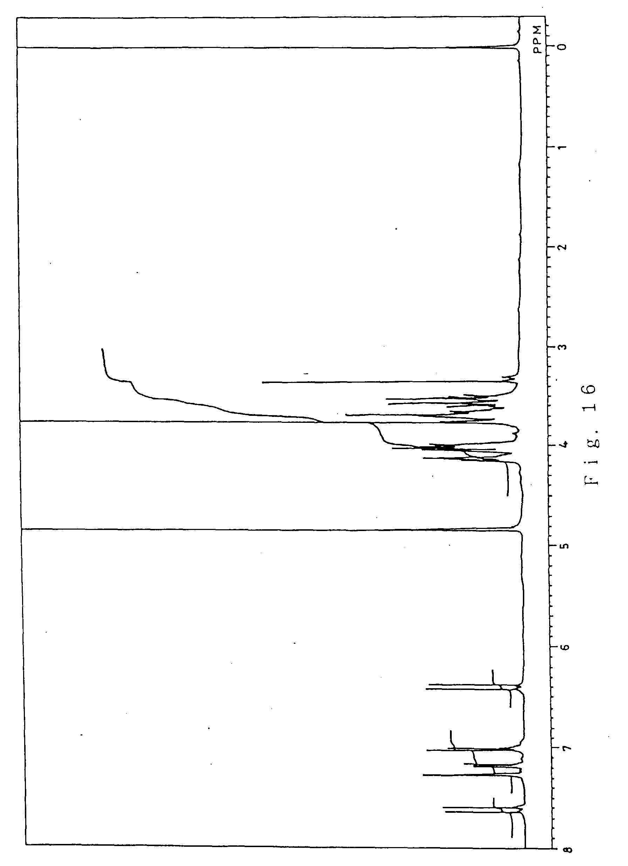

The result is shown in the Fig. 16.

### Example 10 Preparation of the adduct of cinnamic acid and glycerin (6) according to a reaction using an alkaline catalyst

10 g of caffeic acid were dissolved in 90 ml of DMSO, and 100 mg of sodium hydroxide were added. The system was heated to 90 °C and 20 g of glycidol were added. Heating and agitation were carried out for 2 hours. The system was cooled, neutralized by adding hydrochloric acid, solvent was removed and the adduct of glycerin was obtained.

The reaction system was fractionated by the column chromatography ushig hyperporous polymer (HIPOROUS RESIN supplied from MITSUBISHI KASEI KOGYO KABUSHIKI KAISHA), distilled water as a first eluent and a mixture of ethyl alcohol and distilled water with the mixing ratio of 3:7 as a second eluent. The effluent fraction of which the mixing ratio of ethyl alcohol and distilled water was 3:7 was evaporated and the adduct of cinnamic acid and glycerin was obtained.

The yield of the adduct of cinnamic acid and glycerin was 15.1 g.

It was identified that the adduct of cinnamic acid and glycerin of the example included glycerin m,p-diglyceroxy cinnamate and the following compound (1).

### (1) Sodium m,p-diglyceryl cinnamate

(y=1, k=2, X=Na)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the caffeic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the caffeic acid part were observed in a range from δ 7.6 to 6.3 ppm, and signals from hydrogen atoms in the glyceryl group were observed in a range from δ 4.1 to 3.3 ppm.

### Example 11 Preparation of the adduct of cinnamic acid and glycerin (7) according to a reaction using an alkaline catalyst

1 g of methyl p-hydroxy cinnamate was dissolved in 10 ml of DMSO, and 112 mg of sodium hydride were added. The system was heated to 90 °C under N₂ gas flow and 2.1 g of glycidol were added gradually. Heating and agitation were carried out for 0.5 hours. The system was cooled, neutralized by adding hydrochloric acid, and the adduct of glycerin was obtained.

The reaction system was fractionated by column chromatography using the hyperporous polymer (HIPOROUS RESIN supplied from MITSUBISHI KASEI KABUSHIKI KAISHA), distilled water as a first eluent and a mixture of ethyl alcohol and distilled water with the mixing ratio of 3:7 as a second solvent. The effluent fraction of which the mixing ratio of ethyl alcohol and distilled water was 3:7 was evaporated and the adduct of cinnamic acid and glycerin was purified.

The yield of the adduct of cinnamic acid and glycerin was 1.5 g.

It was identified that the adduct of cinnamic acid and glycerin of the example included following compounds (1) to (10).

### (1) Methyl p-glyceroxy cinnamate

(y=2, k=1, X=Me)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the caffeic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the caffeic acid part were observed in a range from δ 7.6 to 6.3 ppm, and signals from hydrogen atoms in the glyceryl group were observed in a range from δ 4.1 to 3.3 ppm.

### (2) Methyl p-glyceroxy cinnamate

(y=3, k=1, X=Me)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the cinnamic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the cinnamic acid part were observed in a range from δ 7.6 to 6.3 ppm, and signals from hydrogen atoms in the glyceryl group were observed in a range from δ 4.1 to 3.3 ppm.

### (3) Methyl p-glyceroxy cinnamate

(y=4, k=1, X=Me)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorption due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the cinnamic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the cinnamic acid part were observed in a range from δ 7.6 to 6.3 ppm, and signals from hydrogen atoms in the glyceryl group were observed in the range from δ 4.1 to 3.3 ppm.

### (4) Methyl p-glyceroxy cinnamate

(y=5, k=1, X=Me)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the cinnamic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the cinnamic acid part were observed in a range from δ 7.6 to 6.3 ppm, and signals from hydrogen atoms in the glyceryl group were observed in the range from δ 4.1 to 3.3 ppm.

### (5) Glyceryl p-glyceroxy cinnamate

### (y=3, k=1, X=G)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the cinnamic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CO₃OD as a solvent with JOEL GX-400 supplied from NIPPON DENSHI KABUSHIKI KAISHA at 35 °C. Signals from the cinnamic acid part were observed in a range from δ 7.6 to 6.3 ppm, and signals from hydrogen atoms in the glyceryl group were observed in a range from δ 4.1 to 3.3 ppm.

### (6) Glyceryl p-glyceroxy cinnamate

(y=4, k=1, X=G)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the cinnamic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the cinnamic acid part were observed in a range from δ 7.6 to 6.3 ppm, and signals from hydrogen atoms in the glyceryl group were observed in a range from δ 4.1 to 3.3 ppm.

### (7) Glyceryl p-glyceroxy cinnamate

(y=5, k=1, X=G)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C⁻NMR spectrum was measured by using CD₃OD as a solvent with JOEL GX-400 supplied from NIPPON DENSHI KABUSHIKI KAISHA at 35 °C. Signals from carbon atoms in the cinnamic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the cinnamic acid part were observed in a range from δ 7.6 to 6.3 ppm, and signal from hydrogen atoms in the glyceryl group were observed in a range from δ 4.1 to 3.3 ppm.

### (8) Sodium p-glyceroxy cinnamate

(y=2, k=1, X=Na)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the cinnamic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the cinnamic acid part were observed in a range from δ 7.6 to 6.3 ppm, and signals from hydrogen atoms in the glyceryl group were observed in a range from δ 4.1 to 3.3 ppm.

### (9) Sodium p-glyceroxy cinnamate

(y=3, k=1, X=Na)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the cinnamic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the cinnamic acid part were observed in a range from δ 7.6 to 6.3 ppm, and signal from hydrogen atoms in the glyceryl group were observed in a range from δ 4.1 to 3.3 ppm.

### (10) Sodium p-glyceroxy cinnamate

(y=4, k=1, X=Na)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the cinnamic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the cinnamic acid part were observed in a range from δ 7.6 to 6.3 ppm, and signals from hydrogen atoms in the glyceryl group were observed in a range from δ 4.1 to 3.3 ppm.

The adduct of cinnamic acid and glycerin of the example included methyl p-glyceroxy cinnamate (y=1, k=1, X=Me), glyceryl p-glyceroxy cinnamate (y=1, k=1, X=G), glyceryl p-glyceroxy cinnamate (y=2, k=1, X=G), and sodium p-glyceroxy cinnamate (y=1, k=1, X=Na).

### Example 12 Preparation of the adduct of cinnamic acid and glycerin (7) according to a reaction using an alkaline catalyst

1 g of methyl caffeate was dissolved in 10 ml of DMSO, and 240 mg of sodium hydride were added. The system was heated to 90 °C under N₂ gas flow and 5 g of glycidol were added gradually. Heating and agitation were carried out for 0.5 hours. The system was cooled, neutralized by adding hydrochloric acid, solvent was removed, and the adduct of glycerin was obtained.

The reaction system was fractionated by the column chromatography using the hyperporous polymer (HIPOROUS RESIN supplied from MITSUBISHI KASEI KOGYO KABUSHIKI KAISHA), distilled water as a first eluent and a mixture of ethyl alcohol and distilled water with the mixing ratio of 3:7 as a second eluent. The effluent fraction of which the mixing ratio of ethyl alcohol and distilled water was 3:7 was evaporated and the adduct of cinnamic acid and glycerin was purified.

The yield of the adduct of cinnamic acid and glycerin was 1.8 g.

It was identified that the adduct of cinnamic acid and glycerin of the example included following compounds (1) to (9).

### (1) Methyl m,p-diglyceroxy cinnamate

(y=1, k=2, X=Me)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the caffeic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the caffeic acid part were observed in a range from δ 7.6 to 6.3 ppm, and signals from hydrogen atoms in the glyceryl group were observed in a range from δ 4.1 to 3.3 ppm.

### (2) Methyl m,p-diglyceroxy cinnamate

(y=2, k=2, X=Me)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the cinnamic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the cinnamic acid part were observed in a range from δ 7.6 ppm to 6.3 ppm, and signals from hydrogen atoms in the glyceryl group were observed in a range from δ 4.1 to 3.3 ppm.

### (3) Methyl m,p-diglyceroxy cinnamate

(y=4, k=2, X=Me)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the cinnamic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the caffeic acid part were observed in a range from δ 7.6 to 6.3 ppm, and signals from hydrogen atoms in the glyceryl group were observed in a range from δ 4.1 to 3.3 ppm.

### (4) Glyceryl m,p-diglyceroxy cinnamate

(y=4, k=2, X=G)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the cinnamic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the cinnamic acid part were observed in a range from δ 7.6 to 6.3 ppm, and signals from hydrogen atoms in the glyceryl group were observed in the range of δ 4.1 - 3.3 ppm.

### (5) Glyceryl m,p-diglyceroxy cinnamate

(y=3, k=2, X=G)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the caffeic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the cinnamic acid part were observed in a range from δ 7.6 to 6.3 ppm, and signal from hydrogen atoms in the glyceryl group were observed in a range from δ 4.1 to 3.3 ppm.

### (6) Glyceryl m,p-diglyceroxy cinnamate

(y=4, k=2, X=G)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the cinnamic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the caffeic acid part were observed in a range from δ 7.6 to 6.3 ppm, and signals from hydrogen atoms in the glyceryl group were observed in a range from δ 4.1 to 3.3 ppm.

### (7) Sodium m,p-diglyceroxy cinnamate

(y=2, k=2, X=Na)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the cinnamic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the cinnamic acid part were observed in a range from δ 7.6 to 6.3 ppm, and signals from hydrogen atoms in the glyceryl group were observed in a range from δ 4.1 to 3.3 ppm.

### (8) Sodium m,p-diglyceroxy cinnamate

(y=3, k=2, X=Na)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorption due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the cinnamic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the cinnamic acid part were observed in a range from δ 7.6 to 6.3 ppm, and signals from hydrogen atoms in the glyceryl group were observed in a range from δ 4.1 to 3.3 ppm.

### (9) Sodium m,p-diglyceroxy cinnamate

(y=4, k=2, X=Na)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the cinnamic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the cinnamic acid part were observed in a range from δ 7.6 to 6.3 ppm, and signals from hydrogen atoms in the glyceryl group were observed in a range from δ 4.1 to 3.3 ppm.

### Example 13 Preparation of the adduct of cinnamic acid and glycerin (10) according to a reaction using an alkaline catalyst

1 g of ethyl p-hydroxy cinnamate was dissolved in 10 ml of DMSO, and 125 mg of sodium hydride were added. The system was heated to 90 °C under N₂ gas flow and 2.5 g of glycidol were added gradually. Heating and agitation were carried out for 0.5 hours. The system was cooled, neutralized by adding hydrochloric acid, solvent was removed, and the adduct of glycerin was obtained.

The reaction system was fractionated by the column chromatography using the hyperporous polymer (HIPOROUS RESIN supplied from MITSUBISHI KASEI KABUSHIKI KAISHA), distilled water as a first eluent and a mixture of ethyl alcohol and distilled water with the mixing ratio of 3:7 as a second eluent. The effluent fraction of which the mixing ratio of ethyl alcohol and distilled water was 3:7 was evaporated and the adduct of cinnamic acid and glycerin was purified.

The yield of the adduct of cinnamic acid and glycerin was 2.3 g.

It was identified that the adduct of cinnamic acid and glycerin of the example included following compounds (1) to (5).

### (1) Ethyl p-glyceroxy cinnamate

(y=1, k=1, X=Et)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the cinnamic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the cinnamic acid part were observed in a range from δ 7.6 to 6.3 ppm, and signals from hydrogen atoms in the glyceryl group were observed in a range of δ 4.1 to 3.3 ppm.

### (2) Ethyl p-diglyceroxy cinnamate

(y=2, k=1, X=Et)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the cinnamic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the cinnamic acid part were observed in a range from δ 7.6 to 6.3 ppm, and signals from hydrogen atoms in the glyceryl group were observed in a range of δ 4.1 to 3.3 ppm.

### (3) Ethyl p-triglyceroxy cinnamate

(y=3, k=1, X=Et)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the cinnamic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the cinnamic acid part were observed in a range from δ 7.6 to 6.3 ppm, and signals from hydrogen atoms in the glyceryl group were observed in a range of δ 4.1 to 3.3 ppm.

### (4) Ethyl p-tetraglyceroxy cinnamate

(y=4, k=1, X=Et)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the cinnamic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the cinnamic acid part were observed in a range from δ 7.6 to 6.3 ppm, and signals from hydrogen atoms in the glyceryl group were observed in a range of δ 4.1 to 3.3 ppm.

### (5) Ethyl p-pentaglyceroxy cinnamate

(y=5, k=1, X=Et)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the cinnamic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the cinnamic acid part were observed in a range from δ 7.6 to 6.3 ppm, and signals from hydrogen atoms in the glyceryl group were observed in a range of δ 4.1 to 3.3 ppm.

The adduct of cinnamic acid and glycerin of the example included glyceryl p-glyceroxy cinnamate (y=1, k=1, X=G), glyceryl p-diglyceroxy cinnamate (y=2, k=1, X=G), glyceryl p-triglyceroxy cinnamate (y=3, k=1, X=G), glyceryl p-tetraglyceroxy cinnamate (y=4, k=1, X=G), glyceryl p-pentaglyceroxy cinnamate (y=5, k=1, X=G), sodium p-glyceroxy cinnamate (y=1, k=1, X=Na), sodium p-diglyceroxy cinnamate (y=2, k=1, X=Na), sodium p-triglyceroxy cinnamate (y=3, k=1, X=Na), sodium p-tetraglyceroxy cinnamate (y=4, k=1, X=Na), and sodium p-pentaglyceroxy cinnamate (y=5, k=1, X=Na).

### Example 14 Preparation of the adduct of cinnamic acid and glycerin (13) according to a reaction using an alkaline catalyst

2 g of ethyl caffeate acid were dissolved in 20 ml of DMSO, and 250 mg of sodium hydride were added. The system was heated to 90 °C under N₂ gas flow and 5 g of glycidol were added gradually. Heating and agitation were carried out for 0.5 hours. The system was cooled, neutralized by adding hydrochloric acid, solvent was removed, and the adduct of glycerin was obtained.

The reaction system was fractionated by the column chromatography using hyper porous polymer (HIPOROUS RESIN supplied from MITSUBISHI KASEI KOGYO KABUSHIKI KAISHA), distilled water as a first eluent and a mixture of ethyl alcohol and distilled water with the mixing ratio of 3:7 as a second eluent. The effluent fraction of which the mixing ratio of ethyl alcohol and distilled water was 3:7 was evaporated and the adduct of cinnamic acid and glycerin was purified.

The yield of the adduct of cinnamic acid and glycerin was 2.3 g.

It was identified that the adduct of cinnamic acid and glycerin of the example included following compounds (1) to (5).

### (1) Ethyl m,p-diglyceroxy cinnamate

(y=1, k=2, X=Et)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the caffeic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the caffeic acid part were observed in a range from δ 7.6 to 6.3 ppm, and signals from hydrogen atoms in the glyceryl group were observed in a range of δ 4.1 to 3.3 ppm.

### (2) Ethyl m,p-diglyceroxy cinnamate

(y=2, k=2, X=Et)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the caffeic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the caffeic acid part were observed in a range from δ 7.6 to 6.3 ppm, and signals from hydrogen atoms in the glyceryl group were observed in a range of δ 4.1 to 3.3 ppm.

### (3) Ethyl m,p-diglyceroxy cinnamate

(y=3, k=2, X=Et)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the caffeic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the caffeic acid part were observed in a range from δ 7.6 to 6.3 ppm, and signals from hydrogen atoms in the glyceryl group were observed in a range of δ 4.1 to 3.3 ppm.

### (4) Ethyl m,p-diglyceroxy cinnamate

(y=4, k=2, X=Et)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the caffeic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the caffeic acid part were observed in a range from δ 7.6 to 6.3 ppm, and signals from hydrogen atoms in the glyceryl group were observed in a range of δ 4.1 to 3.3 ppm.

### (5) Ethyl m,p-diglyceroxy cinnamate

(y=5, k=2, X=Et)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the caffeic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the caffeic acid part were observed in a range from δ 7.6 to 6.3 ppm, and signals from hydrogen atoms in the glyceryl group were observed in a range of δ 4.1 to 3.3 ppm.

The adduct of cinnamic acid and glycerin of the example included glyceryl m,p-diglyceroxy cinnamate (y=1, k=2, X=G), glyceryl m,p-diglyceroxy cinnamate (y=2, k=2, X=G), glyceryl m,p-diglyceroxy cinnamate (y=3, k=2, X=G), glyceryl m,p-diglyceroxy cinnamate (y=4, k=2, X=G), glyceryl m,p-diglyceroxy cinnamate (y=5, k=2, X=G), sodium m,p-diglyceroxy cinnamate (y=1, k=2, X=Na), sodium m,p-diglyceroxy cinnamate (y=2, k=2, X=Na), sodium m,p-diglyceroxy cinnamate (y=3, k=2, X=Na), sodium m,p-diglyceroxy cinnamate (y=4, k=2, X=Na), and sodium m,p-diglyceroxy cinnamate (y=5, k=2, X=Na).

### Example 15 Preparation of the adduct of cinnamic acid and glycerin (14) according to a reaction using an alkaline catalyst

6 g of methyl caffeate were dissolved in 60 ml of DMSO, and 750 mg of sodium hydride were added. The system was heated to 90 °C under N₂ gas flow and 15 g of glycidol were added gradually. Heating and agitation were carried out for 0.5 hours. The system was cooled, neutralized by adding hydrochloric acid, solvent was removed and the adduct of glycerin was obtained.

The reaction system was fractionated by the column chromatography using hyperporous polymer (HIPOROUS RESIN supplied from MITSUBISHI KASEI KABUSHIKI KAISHA), distilled water as a first eluent and a mixture of ethyl alcohol and distilled water with the mixing ratio of 3:7 as a second eluent. The effluent fraction of which the mixing ratio of ethyl alcohol and distilled water was 3:7 was evaporated and the adduct of glycerin was obtained.

The yield of the adduct of cinnamic acid and glycerin was 2.3 g.

It was identified that the adduct of cinnamic acid and glycerin of the example included the following compounds (1) to (5).

### (1) Methyl m,p-diglyceroxy cinnamate

(y=1, k=2, X=Mt)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the caffeic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the caffeic acid part were observed in a range from δ 7.6 to 6.3 ppm, and signals from hydrogen atoms in the glyceryl group were observed in a range of δ 4.1 to 3.3 ppm.

### (2) Methyl m,p-diglyceroxy cinnamate

(y=2, k=2, X=Me)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the caffeic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the caffeic acid part were observed in a range from δ 7.6 to 6.3 ppm, and signals from hydrogen atoms in the glyceryl group were observed in a range of δ 4.1 to 3.3 ppm.

### (3) Methyl m,p-diglyceroxy cinnamate

(y=3, k=2, X=Me)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the caffeic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the caffeic acid part were observed in a range from δ 7.6 to 6.3 ppm, and signals from hydrogen atoms in the glyceryl group were observed in a range of δ 4.1 to 3.3 ppm.

### (4) Methyl m,p-diglyceroxy cinnamate

(y=4, k=2, X=Me)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the caffeic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the caffeic acid part were observed in a range from δ 7.6 to 6.3 ppm, and signals from hydrogen atoms in the glyceryl group were observed in a range of δ 4.1 to 3.3 ppm.

### (5) Methyl m,p-diglyceroxy cinnamate

(y=5, k=2, X=Me)

### ① INFRARED ABSORPTION SPECTROSCOPY

The infrared absorption spectrum of the pure substance was measured by using the IRA-1 infrared absorption spectrometer supplied from NIHON BUNKO KABUSHIKI KAISHA. Absorptions due to the stretching vibration of the hydroxyl group at 3350 cm⁻¹, the stretching vibration of the glyceroxy group at 2900cm⁻¹, and the stretching vibration of the carbonyl group at 1690 cm⁻¹ were observed.

### ② ¹³C-NMR SPECTROSCOPY

The ¹³C-NMR spectrum was measured by using CD₃OD as a solvent with JOEL GX-400 supplied from NIPPON DENSHI KABUSHIKI KAISHA at 35 °C. Signals from carbon atoms in the caffeic acid part were observed in a range from 169 ppm to 103 ppm, and signals from carbon atoms in the glyceryl group were observed in a range from 64 ppm to 43 ppm.

### ③ ¹H-NMR SPECTROSCOPY

The ¹H-NMR spectrum was measured by using CD₃OD as a solvent with JOEL GX-400 supplied from NIPPON DENSHI KABUSHIKI KAISHA at 35 °C. Signals from the caffeic acid part were observed in a range from δ 7.6 to 6.3 ppm, and signals from hydrogen atoms in the glyceryl group were observed in a range of δ 4.1 to 3.3 ppm.

The adduct of cinnamic acid and glycerin of the example included glyceryl m,p-diglyceroxy cinnamate (y=1, k=2, X=G) glyceryl m,p-diglyceroxy cinnamate (y=2, k=2, X=G), glyceryl m,p-diglyceroxy (y=3, k=2, X=G), glyceryl m,p-diglyceroxy cinnamate (y=4, k=2, X=G), glyceryl m,p-diglyceroxy cinnamate (y=5, k=2, X=G), sodium m,p-diglyceroxy cinnamate (y=1, k=2, X=Na), sodium m,p-diglyceroxy cinnamate (y=2, k=2, X=Na), sodium m,p-diglyceroxy cinnamate (y=3, k=2, X=Na), sodium m,p-diglyceroxy cinnamate (y=4, k=2, X=Na), and sodium m,p-diglyceroxy cinnamate (y=5, k=2, X=Na).

Effects of external preparations for skin including above mentioned adduct of cinnamic acid and glycerin will be explained hereinafter.

### Experiment 1 Skin Lotion

According to the formulation as shown in table 1, Application Example 1 represents a skin lotion which was prepared with the adduct of cinnamic acid and glycerin designated as sample 1 (see Example 1), and control 1 represents a skin lotion which was prepared with sodium 2-hydroxy-4-methoxybenzophenone-5-sulfonate.

**TABLE 1**

| COMPONENT | Application EXAMPLE 1 | CONTROL 1 |
|---|---|---|
| A. (ALCOHOL PHASE) | | |
| Ethanol | 5.0 | 5.0 |
| POE oleyl alcohol ether | 2.0 | 2.0 |
| Perfume | q.s. | q.s. |

| B. (AQUEOUS PHASE) | | |
|---|---|---|
| 1,3-butylene glycol | 5.0 | 5.0 |
| Sample 1 | 8.0 | - |
| Sodium 2-hydroxy-4-methoxy benzophenone-5-sulfonate | - | 8.0 |
| Triethanol amine | 0.1 | 0.1 |
| Carboxy vinyl polymer | 0.15 | 0.15 |
| Distilled water | Residual | Residual |

The alcohol phase A was added to the aqueous phase B, and a perfume was made water soluble to obtain a skin lotion.

In Application Example 1, a skin lotion which had no color, was transparent and had a viscosity was obtained, while in Control 1 a lotion of yellow color and no viscosity was obtained.

### Experiment 2 Test for Anti-suntan Effect

Field tests were carried out on a beach using the 2 types of skin lotions prepared in Experiment 1. In the experiment, two samples were respectively applied to right and left halves of each member of a group consisting of 20 men and 20 women, and the degree of sun burning was determined. The criterion for the determination was as follows.

Criterion for evaluation of sun-burning degree

| | |
|---|---|
| Remarkable erythema recognized | × |
| Slight erythema recognized | Δ |
| Erythema not recognized | ○ |

The results are shown in Table 2.

**TABLE 2**

| | APPLIED SECTION of Application EXAMPLE 1 | APPLIED SECTION of CONTROL 1 | |
|---|---|---|---|
| ○ | 35 | 6 | |
| Δ | 5 | 12 | |
| × | 0 | 22 | |
| NUMBER OF SKIN | NONE | ITCHINESS | 10 CASES |
| TROUBLE CASES | | ERUPTION | 2 CASES |

The above results demonstrate that the skin preparation containing the adduct of cinnamic acid and glycerin according to the present invention is more effective for protection from ultraviolet rays than that containing the prior art types of water soluble ultraviolet ray absorbent and has higher safety without causing any skin trouble.

### Experiment 3 Moisture Holding Properties

Changes of skin conductance in a group consisting of 15 men and 15 women were measured under the environmental condition of the room temperature of 25 °C and the relative humidity of 50 %. The skin lotion produced in Experiment 1 was applied to an arm of each member of the group, and the skin conductance of the arm skin was measured 24 hours after the treatment. The moisture holding properties were determined according to the increase ratio. Results of the determination were as follows.$\text{Increase rate of conductance} \text{=} \frac{\text{Increase ratio of conductance value}}{\text{Conductance value before treatment}}$

Criterion for the capability to preserve humidity

| | |
|---|---|
| Increase ratio of conductance : less than 15 % | × |
| Increase ratio of conductance : 15 % to 30 % | Δ |
| Increase ratio of conductance : 30 % or more | ○ |

The results are as shown in Table 3.

**TABLE 3**

| | APPLIED SECTION of Application EXAMPLE 1 | APPLIED SECTION of CONTROL 1 |
|---|---|---|
| ○ | 21 | 6 |
| Δ | 9 | 8 |
| × | 0 | 16 |

The above results demonstrate that the skin preparation containing the adduct of cinnamic acid and glycerin according to the present invention is more excellent in its moisture holding properties than the skin preparation containing the prior art type of water soluble ultraviolet absorbent.

The examples of skin preparations according to the invention will be explained hereinafter. These skin preparations had excellent ultraviolet shielding effects.

### Example 16 Cream

### A. Oily phase

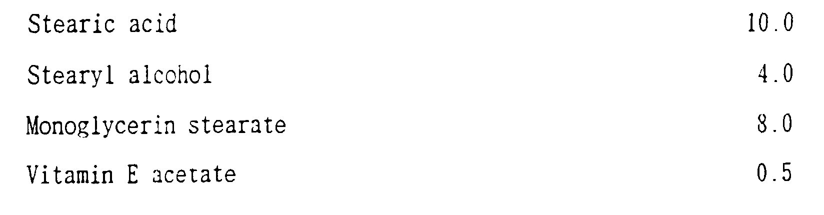

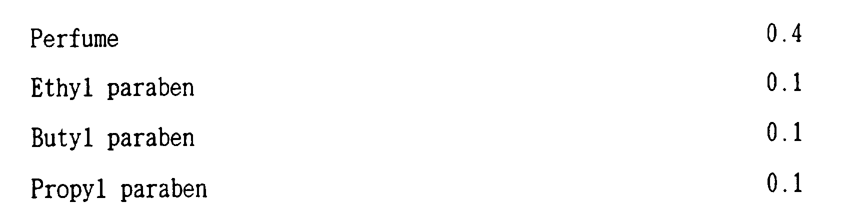

### B. Aqueous phase

| | |
|---|---|
| Propylene glycol | 8.0 |
| Glycerin | 2.0 |
| Glyceryl p-glyceroxy cinnamate | 6.0 |
| Potassium hydrate | 0.4 |
| Trisodium edetate | 0.05 |
| Distilled water | Residual |

### <Preparation process>

The oily phase A and the aqueous phase B were heated to 70 °C and completely dissolved respectively. Then the phase A was added to the phase B, and the mixture was emulsified by an emulsifier. Then the emulsion was cooled by a heat exchanger and the cream was obtained.

### Example 17 Cream

### A. Oily phase

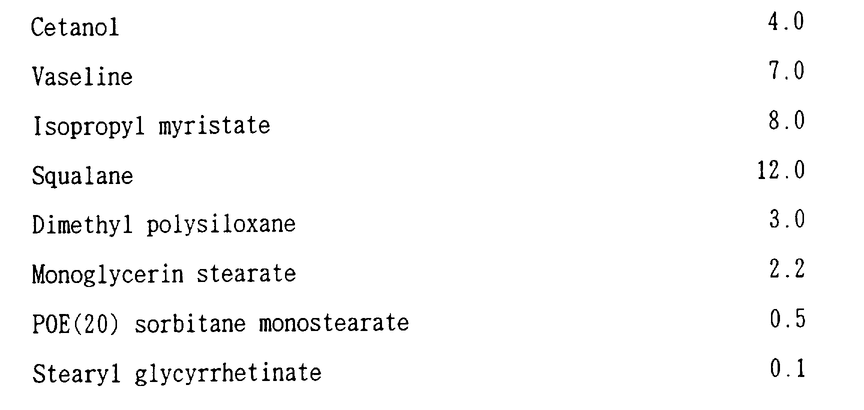

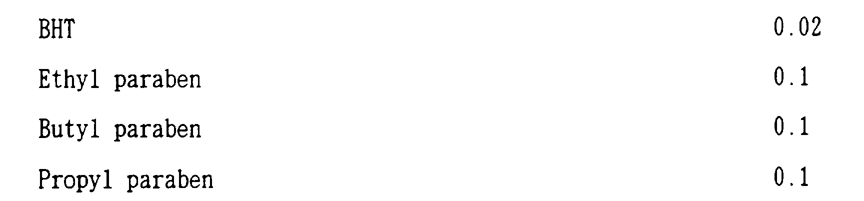

### B. Aqueous phase

| | |
|---|---|
| 1,3 butylene glycol | 7.0 |
| Disodium edetate | 0.07 |
| Phenoxy ethanol | 0.2 |
| Magnesium L-ascorbyl -2- phosphate | 3.0 |
| Polyacrylic acid alkyl ester | 1.0 |
| Methyl p-glyceroxy cinnamate | 8.0 |
| Distilled water | Residual |

### <Preparation process>

The cream was obtained according to the procedure as described in the Example 16.

### Example 18 Skin Lotion with powder

### A. Oily phase

| | |
|---|---|
| Ethanol | 8.0 |
| POE(60) glyceryl monoisostearate | 2.0 |
| L-menthol | 0.1 |
| Camphor | 0.1 |
| Methyl paraben | 0.2 |
| Perfume | q.s. |

### B. Aqueous phase

| | |
|---|---|
| Glycerin | 3.5 |
| Sodium p-glyceroxy cinnamate | 4.0 |
| Zinc oxide | 1.5 |
| Kaolin | 0.5 |
| Bentonite | 0.3 |
| Sodium hexamethaphosphate | 0.03 |
| Distilled water | Residual |

### <Preparation process>

The skin lotion was obtained according to the same procedure as described in Example 16.

## Claims

1. An adduct of cinnamic acid and glycerin represented by the following general formula (I): 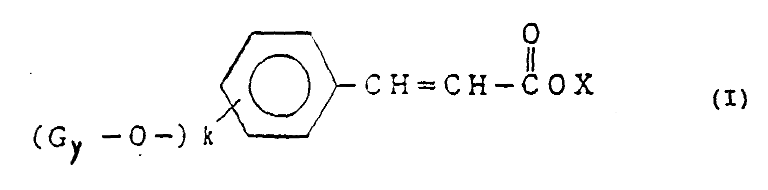 wherein
G represents a glycerin residue;
y represents the average mole number of glycerin residues and is at least 1;
k is 1 to 3; and
X represents hydrogen, an ion, a straight chain or branched chain alkyl group, an unsaturated alkyl group, a cycloalkyl group or G_{y};
provided that the compound of formula (I) is not 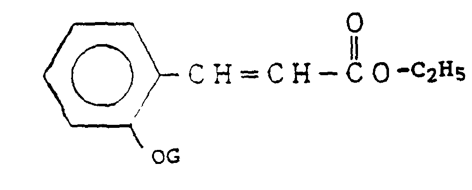 wherein G represents a glycerin residue.

2. An ultraviolet absorbent, containing the adduct of cinnamic acid and glycerin according to claim 1.

3. A skin preparation containing one or more types of the adduct of cinnamic acid and glycerin according to claim 1.

## Patentansprüche

1. Zimtsäure/Glycerol-Addukt der folgenden allgemeinen Formel (I): 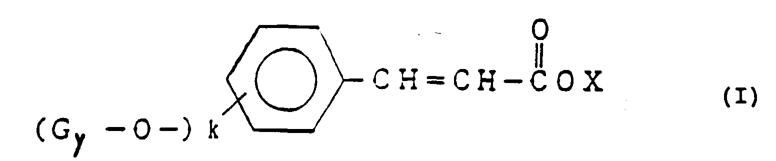 in der
G einen Glycerolrest bedeutet,
y die durchschnittliche Molzahl des Glycerolrests bedeutet und mindestens 1 ist,
k 1 bis 3 bedeutet, und
X ein Wasserstoffatom, ein Ion, einen geradkettigen oder verzweigtkettigen Alkylrest, einen ungesättigten Alkylrest, einen Cycloalkylrest oder G_{y} bedeutet, mit der Maßgabe, daß die Verbindung der Formel (I) verschieden von 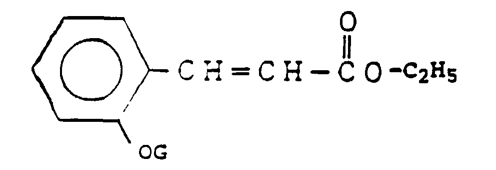 ist, wobei G einen Glycerolrest darstellt.

2. Ultraviolettabsorber, enthaltend das Zimtsäure/Glycerol-Addukt nach Anspruch 1.

3. Dermatologische Präparation, enthaltend eine oder mehrere Arten des Zimtsäure/Glycerol-Adduktes nach Anspruch 1.

## Revendications

1. Produit d'addition de l'acide cinnamique et de la glycérine représenté par la formule générale suivante (I): 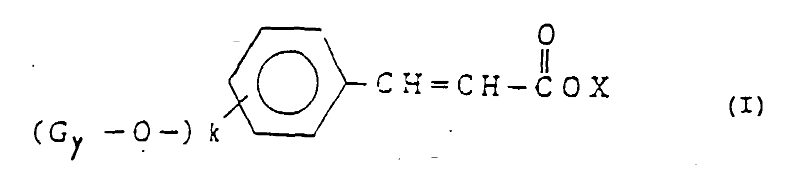 où
G représente un résidu de la glycérine ;
y représente le nombre moyen de moles des résidus de glycérine et il est d'au moins 1 ;
k est de 1à 3 ; et
X représente l'hydrogène, un ion, un groupe alkyle à chaine droite ou ramifiée, un groupe alkyle insaturé, un groupe cycloalkyle ou G_{y} ;
à la condition que le composé de formule (I) ne soit pas 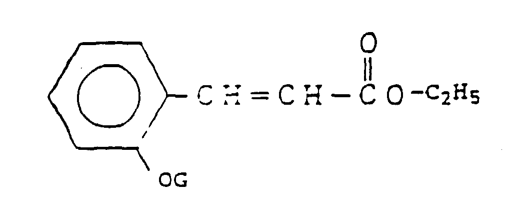 où
G représente un résidu de la glycérine.

2. Absorbeur d'ultraviolet contenant le produit d'addition de l'acide cinnamique et de la glycérine selon la revendication 1.

3. Préparation pour la peau contenant un ou plusieurs types du produit d'addition de l'acide cinnamique et de la glycérine selon la revendication 1.
